# EUROPEAN PATENT APPLICATION

(11) **EP 3 081 642 A1**
(43) Date of publication of application: **19.10.2016**
(21) Application number: 14866146.5
(22) Date of filing: 27.11.2014
(51) Int. Cl.: C12N 15/09, C07K 14/435, C07K 19/00, C12N 1/15, C12N 1/19, C12N 1/21, C12N 5/10, G01N 33/68

(54) **NOVEL FLUORESCENT PROTEIN**

(30) Priority: 28.11.2013 JP 2013246642
(71) Applicant: Kyoto Sangyo University, Kyoto-shi, Kyoto 603-8555 (JP); Horiba, Ltd., Kyoto-shi, Kyoto 601-8510 (JP)
(72) Inventor: NAKAYAMA, Hideki, Kyoto-shi Kyoto 603-8555 (JP); SHIMAMOTO, Nobuo, Kyoto-shi Kyoto 603-8555 (JP); TAKAHASHI, Mayako, Kyoto-shi Kyoto 601-8510 (JP)
(74) Representative: Isarpatent
(86) International application number: PCT/JP2014/081308
(87) International publication number: WO 2015/080178

(57) **Abstract**

The present invention provides a fluorescent protein and the like containing an amino acid sequence the same or substantially the same as the amino acid sequence shown in SEQ ID NO: 2.

## Description

### Technical Field

The present invention relates to a novel fluorescent protein, a nucleic acid encoding the fluorescent protein, an expression vector, a transformant, and a production method of the fluorescent protein, comprising culturing the transformant.

### Background Art

Green Fluorescent Protein (GFP) is a protein that became also known socially after Osamu Shimomura, Marty Chalfy, Roger Tsien were awarded the Nobel Prize in Chemistry in 2008. The protein was found by Mr. Shimomura in 1979 as a fluorescent protein that Aequorea victoria, which emits fluorescence, has.
In 1994, moreover, Chalfy et al. found that Escherichia coli and nematode produce a fluorescent protein by merely cloning the GFP gene, and that GFP can be used as a useful tool in the cell biology. Thereafter, it was clarified that GFP emits fluorescence simply by being oxidized, and Tsien et al. developed useful GFP by introducing many mutations and clarified the mechanism of fluorescence emission.

At present, fluorescent proteins having a similar structure have been found in various luminescent organisms such as coral, eel and the like. A fusion protein obtained by fusing a gene encoding same with a target gene has enabled optical observation of the distribution and behavior of a protein encoded by the target gene, as well as observation of the intracellular pH and redox potential. In this way, many GFPs and analogs thereof are used at present as reporter molecules.

GFP and analogs thereof have a generic term of Fluorescent Protein (FP). FP has a distinct structure in which several amino acid residues constituting a fluorescent group are enclosed in a cylindrical structure surrounded by β sheet called β can. For example, in Aequorea victoria GFP, a fluorescent group is constituted of an amino acid consisting of Ser65-Tyr66-Gly67. That is, after translation of FP mRNA as a polypeptide, 1) a β can structure is formed (structure formation also occurs partly during translation), and 2) the β can structure is oxidized to form a fluorescent group. The formation processes of fluorescent groups of various FPs are described in non-patent documents 1 - 3.

The intensity of fluorescence from one molecule of fluorescent group is in proportion to "molar absorption coefficient x quantum efficiency" of a fluorescent group. The molar absorption coefficient and quantum efficiency of almost all FPs show a small difference in terms of order and only about several times. Therefore, the fluorescence intensity of FP in the cell strongly relies on the number and concentration of mature fluorescent group rather than the kind of FP. Firstly, the absolute amount of FP as an intracellular protein in the expression stationary phase is determined by the rate of expression and structure formation, and the decomposition rate. Super Folder GFP has been reported (non-patent document 4) as one of the FPs having strong fluorescence, and the protein has a codon selected to provide an optimal unwinding of the polypeptide chain after translation (β can structure formation). This proves that the unwinding rate is related to the fluorescence intensity of FP. When the unwinding is fast to a certain degree, however, formation of the fluorescent group becomes the factor for determining the fluorescence intensity of FP. Since the fluorescent group can be formed by an oxidation reaction in every case, the cells are desirably present in an oxidation environment so that FP will produce fluorescence. Nevertheless, cells under active division are in a reduction environment, in which the formation of fluorescent group may be delayed. It is well known that FP expressed in Escherichia coli is dark in the growth phase and becomes bright in the stationary phase. The modified GFP (Ser65 substituted by Thr65) produced by Tsien shows a fast process of fluorescent group formation, as a result of which the modified GFP becomes bright. This proves that the formation of fluorescent group is related to the fluorescence intensity of FP.

When excitation is continued, all fluorescent groups sooner or later undergo a photochemical reaction to result in discoloration (photo-bleaching). In most cases, the photochemical reaction is an irreversible reaction, and is assumed to be one kind of photooxidation reaction (non-patent documents 1, 5, 6). To prevent photo-bleaching in the case of an organic dye, for example, some means needs to be adopted to prevent contact with an oxygen molecule by embedding the organic dye in a resin and the like. However, such means is not possible for FP, since FP can maintain the structure only in an aqueous solution. When compared to organic dyes, FP does not permit easy photo-bleaching since the fluorescent group is protected by a β can structure. However, it is still photo-bleached in about several seconds. In an experiment using a fluorescence microscope, since a focusing operation required several seconds, many FPs were photo-bleached during the operation, which was inconvenient for fluorescence observation. As the situation stands, therefore, the development of FP which is slowly photo-bleached in vitro and in vivo, and has strong fluorescence intensity, has been desired.

### [Document List]

### [non-patent documents]

non-patent document 1: Zimmer, M., Green Fluorescent Protein (GFP): Applications, Structure, and Related Photophysical Behavior. Chem. Rev.2002, 102, 759-781.
non-patent document 2: Mizuno, H. et al., Photo-Induced Peptide Cleavage in the Green-to-Red Conversion of a Fluorescent Protein. Mol. Cell 2003, 12, 1051-1058.
non-patent document 3: Bourgeois, D. et al., Reversible Photoswitching in Fluorescent Proteins: a Mechanistic View. IUBMB Life 2012, 64, 482-491.
non-patent document 4: Pedelacq, J.-D. et al., Engineering and Characterization of a Superfolder Green Fluorescent Protein. Nat. Biotechnol.2005, 24, 79-88.
non-patent document 5: Ito, Y. et al., A Novel Mutant of Green Fluorescent Protein with Enhanced Sensitivity for Microanalysis at 488 Nm Excitation. Biochem. Biophys. Res. Commun. 1999, 264, 556-560.
non-patent document 6: Nagai, T. et al., A Variant of Yellow Fluorescent Protein with Fast and Efficient Maturation for Cell-Biological Applications. Nat. Biotechnol. 2002, 20, 87-90.

### SUMMARY OF THE INVENTION

### Problems to be Solved by the Invention

The present invention aims to provide a novel fluorescent protein superior to conventional FP in photo-bleaching time and fluorescence intensity.

### Means of Solving the Problems

In the process of developing GFP having sensitivity to redox potential, and strong fluorescence intensity, the present inventors accidentally found a novel fluorescent protein having characteristics absent in other GFP. For example, the novel fluorescent protein has the following characteristics. (1) remarkable resistance to photo-bleaching, (2) maximum excitation wavelength of 493 nm, maximum fluorescence wavelength of 513 nm, (3) 12 nm longer maximum fluorescence wavelength during dimer formation as compared to monomer, (4) brightness equivalent to known GFP with strong fluorescence intensity such as GFP-UV4 and Venus, and the like.

The present inventors have conducted further studies based on these findings and completed the present invention.

That is, the present invention provides
[1] a fluorescent protein comprising an amino acid sequence the same or substantially the same as the amino acid sequence shown in SEQ ID NO: 2;
[2] a nucleic acid comprising a base sequence encoding the fluorescent protein of [1];
[3] an expression vector comprising the nucleic acid of [2];
[4] a transformant comprising the expression vector of [3];
[5] a fusion protein comprising the fluorescent protein of [1] and other protein;
[6] a method of detecting localization or dynamics of the fusion protein of [5] in a cell, comprising measuring the position of fluorescence of the fusion protein in the cell;
[7] a method of detecting formation of a complex of two or more, the same or different fusion proteins of [5], comprising measuring the maximum fluorescence wavelength of the complex; and the like.

### Effect of the Invention

Since the fluorescent protein of the present invention requires a long time before photo-bleaching and also has strong fluorescence intensity, it facilitates observation of fluorescence in a sample in vivo or in vitro. In addition, the fluorescence of the fluorescent protein of the present invention can be observed with a standard B excitation mirror set, since the maximum excitation wavelength is 493 nm, and the maximum fluorescence wavelength is 513 nm. Furthermore, since the maximum fluorescence wavelength becomes 12 nm longer via dimer formation, the fluorescent protein is expected to be an effective tool for confirming dimer formation of the target molecule.

### Brief Description of the Drawings

Fig. 1 is the base sequence (SEQ ID NO: 4) of modified pTrc promoter (pTrc-do promoter). The single underlined part shows -35 box, -10 box, a transcription initiation site, and a translation initiation site, from the left. The double underlined part shows a modified operator part.
Fig. 2 shows stained gel after separation of 2-mercaptoethanol treated (2-Me(+)) or untreated (2-Me(-)) B-maggio with SDS polyacrylamide gel.
Fig. 3 shows the maximum excitation wavelength (493 nm) and the maximum fluorescence wavelength (513 nm) of B-maggio, as well as the maximum excitation wavelength (510 nm) and the maximum fluorescence wavelength (525 nm) of Twin B-maggio.

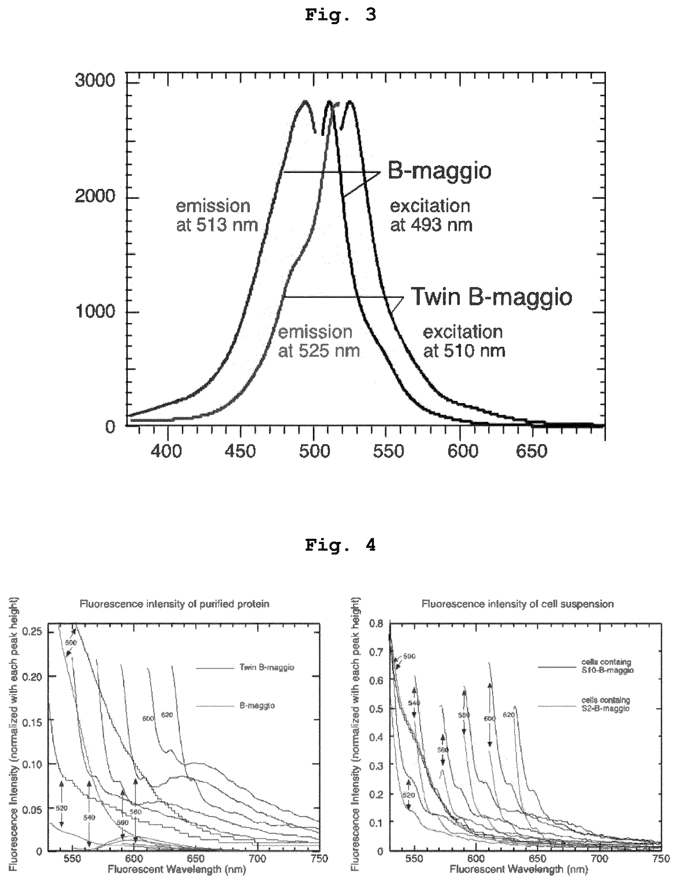

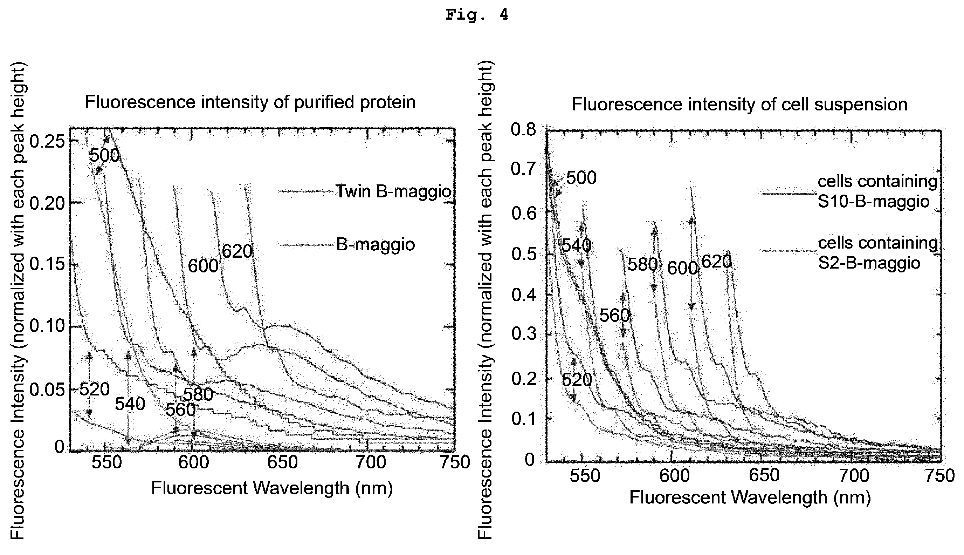

In Fig. 4, the left Figure shows the off-peak fluorescence spectrum on the long wavelength side of B-maggio or Twin B-maggio excited with an excitation light at each wavelength. The right Figure shows the off-peak fluorescence spectrum on the long wavelength side of Escherichia coli mutation strain (S2-B-maggio strain) in which B-maggio gene is inserted into rpsB gene (ribosome protein S2) excited with an excitation light at each wavelength or Escherichia coli mutation strain (S10-B-maggio strain) in which B-maggio gene is inserted into rpsJ gene (ribosome protein S10). The intensity of the fluorescence spectrum is shown by relative intensity when the strongest fluorescence peak on the long wavelength side than the V excitation light side in each sample is 1. The numbers in each Figure show the wavelengths of each excitation light. It is shown that the relative amount of fluorescence at an observation wavelength (>575 nm) after excitation with G excitation light is higher in Twin B-maggio and S10-B-maggio strain than in B-maggio and S2-B-maggio strain.

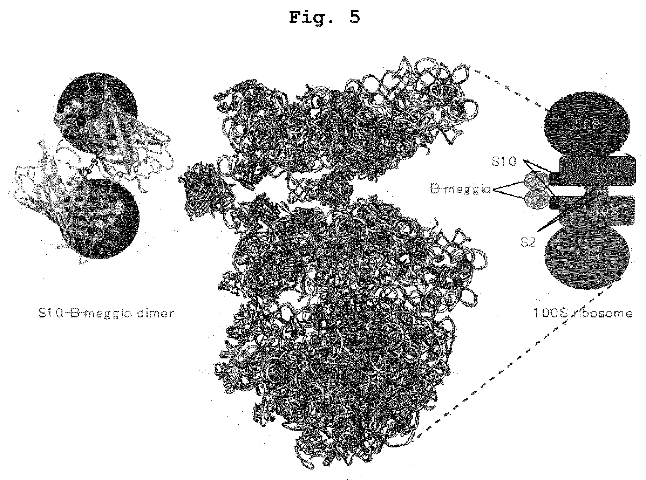

In Fig. 5, the right Figure shows the structure concept of 100S ribosome of E. coli. 70S ribosome is constituted of 30S subunit and 50S subunit, and the dimer thereof becomes 100S ribosome. Two proteins S2, S10 in the 30S subunit are both present on the contact surface of the dimer. When S2 is fused with B-maggio, formation of 100S ribosome is inhibited, whereas it is not inhibited in S10. The center Figure is a reconstitution Figure of a dimer structure of a high resolution ribosome crystal structure (PDB3V22) optimally fitted in a low resolution ribosome, which is obtained by electron microscope tomography and using a cryo-electron microscope method (cryoEM). The left Figure shows spatial configuration of S10 protein fusion B-maggio when 70S ribosome was dimerized. S=S shows the position of the disulfide bond between B-maggio.
Fig. 6 shows optical detection of the fluorescence of S10-B-maggio strain in the growth phase (3 hr after inoculation to medium) or the stationary phase (18 hr after inoculation to medium), by excitation with an excitation light at each wavelength.

### Description of Embodiments

The present invention provides a fluorescent protein containing an amino acid sequence the same or substantially the same as the amino acid sequence shown in SEQ ID NO: 2 (hereinafter to be also referred to as the fluorescent protein of the present invention). The fluorescent protein may be a protein biochemically synthesized in a chemical synthesis or cell-free translation system, or a recombinant protein produced from a transformant introduced with a nucleic acid containing a base sequence encoding the above-mentioned amino acid sequence.

Examples of the amino acid sequence the same or substantially the same as the amino acid sequence shown in SEQ ID NO: 2 include an amino acid sequence having a homology of not less than about 85%, preferably not less than about 90%, further preferably not less than about 95%, most preferably not less than about 98%, with the amino acid sequence shown in SEQ ID NO: 2 and the like. As used herein, the "homology" means the proportion (%) of the same amino acid residues and similar amino acid residues relative to the total overlapping amino acid residues, in an optimal alignment (preferably, the algorithm is capable of considering introduction of gap into one of or both of the sequences for optimal alignment), when two amino acid sequences are aligned using a mathematical algorithm known in the technical field. The "similar amino acid" means amino acids having similar physicochemical properties and, for example, amino acids classified in the same group such as aromatic amino acids (Phe, Trp, Tyr), aliphatic amino acids (Ala, Leu, Ile, Val), polar amino acids (Gln, Asn), basic amino acids (Lys, Arg, His), acidic amino acids (Glu, Asp), amino acids having a hydroxyl group (Ser, Thr), amino acids having a small side chain (Gly, Ala, Ser, Thr, Met) and the like can be mentioned. It is predicted that the substitution with such similar amino acids does not change the phenotype of the protein (that is, conservative amino acid substitution). Specific examples of the conservative amino acid substitution are well known in the technical field and are described in various documents (e.g., Bowie et al., Science, 247: 1306-1310 (1990)).

The homology of the amino acid sequences in the present specification can be calculated using homology calculation algorithm NCBI BLAST (National Center for Biotechnology Information Basic Local Alignment Search Tool) and under the following conditions (expectancy =10; gap allowed; matrix =BLOSUM62; filtering=OFF). Examples of other algorithm for determining the homology of the amino acid sequence include the algorithm described in Karlin et al., Proc. Natl. Acad. Sci. USA, 90: 5873-5877 (1993) [said algorithm is incorporated in the NBLAST and XBLAST program (version 2.0) (Altschul et al., Nucleic Acids Res., 25: 3389-3402 (1997))], the algorithm described in Needleman et al., J. Mol. Biol., 48: 444-453 (1970) [said algorithm is incorporated in the GAP program in the GCG software package], the algorithm described in Myers and Miller, CABIOS, 4: 11-17 (1988) [said algorithm is incorporated in ALIGN program (version2.0) which is a part of the CGC sequence alignment software package], the algorithm described in Pearson et al., Proc. Natl. Acad. Sci. USA, 85: 2444-2448 (1988) [said algorithm is incorporated in the FASTA program in the GCG software package] and the like, and these can also be similarly used preferably.

More preferably, an amino acid sequence substantially the same as the amino acid sequence shown in SEQ ID NO: 2 is an amino acid sequence having identity of not less than about 85%, preferably not less than about 90%, further preferably not less than about 95%, most preferably not less than about 98%.

As the protein containing an amino acid sequence substantially the same as the amino acid sequence shown in SEQ ID NO: 2, for example, a protein containing an amino acid sequence substantially the same as the aforementioned amino acid sequence shown in SEQ ID NO: 2 and having properties of substantially the same quality as those of a protein containing the amino acid sequence shown in SEQ ID NO: 2 and the like is preferable. As used herein, the "property" refers to photo-bleaching time, fluorescence intensity, maximum excitation wavelength, maximum fluorescence wavelength, a shift of maximum excitation wavelength and maximum fluorescence wavelength, which is associated with dimer formation, and the like. As for the shift of maximum excitation wavelength and maximum fluorescence wavelength, which is associated with dimer formation, more particularly, a fluorescent protein containing the amino acid sequence shown in SEQ ID NO: 2 has a maximum excitation wavelength of 493 nm and a maximum fluorescence wavelength of 513 nm in the case of a monomer, and a maximum excitation wavelength of 510 nm, and a maximum fluorescence wavelength of 525 nm in the case of a dimer. Therefore, when the fluorescent proteins of the present invention formed a complex containing a dimer, the maximum excitation wavelength becomes longer by 17 nm, and the maximum fluorescence wavelength becomes longer by 12 nm, as compared to a monomer. In addition, "properties of substantially the same quality" means that the properties thereof are qualitatively (e.g., biochemically or physiologically) the same. Therefore, the aforementioned properties are preferably of the same quality. The level of these properties may be different (e.g., photo-bleaching time and fluorescence intensity fall within about 0.1 - about 10-fold, preferably about 0.5 - about 2-fold. The maximum excitation wavelength, and the maximum fluorescence wavelength fall within ±10 nm, preferably ±5 nm. The shift in the maximum excitation wavelength falls within about 10 nm - about 25 nm, preferably about 15 nm - about 20 nm. The shift in the maximum fluorescence wavelength falls within about 5 nm - about 20 nm, preferably about 10 nm - about 15 nm).

The measurement of the aforementioned properties can be performed according to a method known per se.

In addition, the fluorescent protein of the present invention also encompasses, for example, an amino acid sequence wherein one or more (preferably, 1 - about 50, more preferably 1 - about 10, further preferably 1 - several (2, 3, 4 or 5)) amino acids in the amino acid sequence shown in SEQ ID NO: 2 are substituted by other amino acids. When the amino acid sequence is substituted, the position of substitution is not particularly limited as long as the aforementioned properties of the fluorescent protein of the present invention are maintained. However, it is preferably the 48th amino acid (Ser), the 64th amino acid (Leu), the 65th amino acid (Thr), the 153rd amino acid (Thr), the 163rd amino acid (Ala), the 204th amino acid (Cys), the 205th amino acid (Ser), the 206th amino acid (Ala), the 207th amino acid (Leu) or the 208th amino acid (Ser) in the amino acid sequence shown in SEQ ID NO: 2, and more preferably the 48th amino acid (Ser), the 64th amino acid (Leu), the 65th amino acid (Thr), the 153rd amino acid (Thr), the 163rd amino acid (Ala) or the 204th amino acid (Cys) in the amino acid sequence shown in SEQ ID NO: 2. More particularly, therefore, the fluorescent protein of the present invention encompasses a protein wherein at least one amino acid selected from the group consisting of the 48th amino acid (Ser), the 64th amino acid (Leu), the 65th amino acid (Thr), the 153rd amino acid (Thr), the 163rd amino acid (Ala), the 204th amino acid (Cys), the 205th amino acid (Ser), the 206th amino acid (Ala), the 207th amino acid (Leu) and the 208th amino acid (Ser) in the amino acid sequence shown in SEQ ID NO: 2 (preferably, the 48th amino acid (Ser), the 64th amino acid (Leu), the 65th amino acid (Thr), the 153rd amino acid (Thr), the 163rd amino acid (Ala) and the 204th amino acid (Cys) in the amino acid sequence shown in SEQ ID NO: 2) is substituted, and optionally encompasses a protein having properties of substantially the same quality as a protein having the amino acid sequence shown in SEQ ID NO: 2.

Alternatively, the position of substitution in the amino acid sequence may be the 146th amino acid (Asn) or the 147th amino acid (Ser) in the amino acid sequence shown in SEQ ID NO: 2. In this case, the 204th Cys in the amino acid sequence shown in SEQ ID NO: 2 is preferably substituted by Gln. More particularly, therefore, the fluorescent protein of the present invention encompasses a protein wherein at least one amino acid from the 146th amino acid (Asn) and the 147th amino acid (Ser) in the amino acid sequence shown in SEQ ID NO: 2 is substituted, and optionally encompasses a protein having properties of substantially the same quality as a protein having the amino acid sequence shown in SEQ ID NO: 2. Particularly, a protein wherein the 204th Cys is substituted by Gln and the 147th Ser is substituted by Cys in the amino acid sequence shown in SEQ ID NO: 2 shows high photosensitivity to a shift in the maximum fluorescence wavelength due to dimer formation. As an amino acid used for substitution, the above-mentioned "similar amino acid" is preferably used.

Other than the above, the fluorescent protein of the present invention also encompasses, for example, a protein containing (1) an amino acid sequence wherein one or more (preferably, 1 - about 100, more preferably 1 - about 50, further preferably 1 - about 10, particularly preferably 1 - several (2, 3, 4 or 5)) amino acids in the amino acid sequence shown in SEQ ID NO: 2 are deleted, (2) an amino acid sequence wherein one or more (preferably, 1 - about 100, more preferably 1 - about 50, further preferably 1 - about 10, particularly preferably 1 - several (2, 3, 4 or 5)) amino acids are added to the amino acid sequence shown in SEQ ID NO: 2, (3) an amino acid sequence wherein one or more (preferably, 1 - about 50, more preferably 1 - about 10, further preferably 1 - several (2, 3, 4 or 5)) amino acids are inserted into the amino acid sequence shown in SEQ ID NO: 2 or (4) an amino acid sequence which is a combination thereof or the like.

As mentioned above, when the amino acid sequence is inserted or deleted, the position of insertion or deletion is not particularly limited as long as the properties of the fluorescent protein of the present invention are maintained.

The fluorescent protein of the present invention can be produced according to a known peptide synthesis method. The peptide synthesis method may be any of, for example, solid phase synthesis process and solution phase synthesis process. The object fluorescent protein can be produced by condensing a partial peptide or amino acid capable of constituting the fluorescent protein of the present invention and the remaining portion and, when the resultant product has a protecting group, removing the protecting group. Here, the condensation and removal of the protecting group are performed according to a method known per se, for example, the methods described in the following (1) and (2).
(1) M. Bodanszky and M. A. Ondetti, Peptide Synthesis, Interscience Publishers, New York (1966)
(2) Schroeder and Luebke, The Peptide, Academic Press, New York (1965)

The thus-obtained the fluorescent protein of the present invention can be purified and isolated by a known purification method. Examples of the purification method here include solvent extraction, distillation, column chromatography, liquid chromatography, recrystallization, a combination of these and the like.

When the fluorescent protein of the present invention obtained by the above-mentioned method is a free form, the free form can be converted to a suitable salt by a known method or a method analogous thereto. Conversely, when the fluorescent protein of the present invention is obtained as a salt, the salt can be converted to a free form or other salt by a known method or a method analogous thereto.

Furthermore, the fluorescent protein of the present invention can also be produced by culturing a transformant containing a nucleic acid encoding same, and separating and purifying the fluorescent protein from the resulting culture.
The nucleic acid encoding the fluorescent protein of the present invention may be DNA or RNA, or may be DNA/RNA chimera. Preferred is DNA. In addition, the nucleic acid may be double stranded or single stranded. When it is double stranded, it may be a double stranded DNA, a double stranded RNA or a DNA:RNA hybrid. When it is single stranded, it may be a sense strand (i.e., coding strand), or an antisense strand (i.e., non-coding strand).

As DNA encoding the fluorescent protein of the present invention, synthetic DNA and the like can be mentioned. For example, it can be acquired by converting, according to a method known per se such as ODA-LA PCR method, Gapped duplex method, Kunkel method and the like, or a method analogous thereto, and by using a known kit, for example, Mutan™-super Express Km (TAKARA BIO INC.), Mutan™-K (TAKARA BIO INC.) and the like, a full-length GFP cDNA (e.g., nucleotide No. 289-1005 of GenBank Accession No. U17997 and the like), which was directly amplified by Reverse Transcriptase-PCR (hereinafter abbreviated as "RT-PCR method") by using total RNA or mRNA fraction prepared from a cell or tissue derived from Aequorea victoria as a template. Alternatively, it can also be acquired by converting, according to the above-mentioned method, a cDNA cloned from a cDNA library, prepared by inserting a fragment of the above-mentioned total RNA or mRNA into a suitable vector, by colony or plaque hybridization method or PCR method and the like. The vector used for the library may be any such as bacteriophage, plasmid, cosmid, phagemid and the like.

Examples of the DNA encoding the fluorescent protein of the present invention include a DNA containing a base sequence the same as or substantially the same as the base sequence shown in SEQ ID NO: 1 and the like.

Examples of the DNA containing a base sequence substantially the same as the base sequence shown in SEQ ID NO: 1 include (1) a DNA containing a base sequence which is the base sequence shown in SEQ ID NO: 1 having a mutation that does not change the amino acid sequence shown in SEQ ID NO: 2 (silent mutation) or (2) a DNA containing a base sequence having a homology of not less than about 85%, preferably not less than about 90%, further preferably not less than about 95%, most preferably not less than about 98%, with the base sequence shown in SEQ ID NO: 1, and encoding a protein having properties of substantially the same quality as those of the aforementioned fluorescent protein of the present invention, and the like.

The homology of the base sequences in the present specification can be calculated using homology calculation algorithm NCBI BLAST (National Center for Biotechnology Information Basic Local Alignment Search Tool) and under the following conditions (expectancy =10; gap allowed; filtering=ON; match score=1; mismatch score=-3). Other algorithm for determining the homology of the base sequence is similarly exemplified preferably by the above-mentioned homology calculation algorithm for amino acid sequence.

The DNA encoding the fluorescent protein of the present invention can be, as mentioned above, cloned by amplifying full-length GFP cDNA, which was directly amplified by RT-PCR method by using total RNA or mRNA derived from Aequorea victoria as a template, according to the above-mentioned method by using a synthetic DNA primer having a desired mutation, which is a part of the base sequence of the cDNA.

An expression vector containing a DNA encoding the fluorescent protein of the present invention can be produced, for example, by cleaving out a DNA fragment encoding the aforementioned fluorescent protein, and ligating the DNA fragment at the downstream of a promoter in a suitable expression vector. Alternatively, as described in the below-mentioned Examples, an expression vector containing a DNA encoding the fluorescent protein of the present invention can be obtained by mixing DNA fragments containing an expression vector obtained by amplification using the expression vector (SEQ ID NO: 3) containing a DNA encoding GFPUV as a template and primers of SEQ ID NOs: 5 - 12, and directly transforming Escherichia coli with the fragment amplified using a primer set of SEQ ID NOs: 5 and 6.

As the expression vector, plasmid derived from Escherichia coli (e.g., pBR322, pBR325, pUC12, pUC13, pTrc); plasmid derived from Bacillus subtilis (e.g., pUB110, pTP5, pC194); plasmid derived from yeast (e.g., pSH19, pSH15); insect cell expressing plasmid (e.g., pFast-Bac); animal cell expressing plasmid (e.g., pSRα, pA1-11, pXT1, pRc/CMV, pRc/RSV, pcDNAI/Neo); bacteriophage such as λphage and the like; insect virus vector such as baculovirus and the like (e.g., BmNPV, AcNPV); animal virus vector such as retrovirus, vaccinia virus, adenovirus and the like, and the like are used.

The promoter may be any as long as it is an appropriate promoter for the host used for gene expression.

For example, when the host is an animal cell, β-actin promoter, SRα promoter, SV40 promoter, LTR promoter, CMV (cytomegalovirus) promoter, RSV (Rous sarcoma virus) promoter, MoMuLV (Moloney murine leukemia virus) LTR, HSV-TK (herpes simplex virus thymidine kinase) promoter, trc promoter, trc modified promoter and the like are used.

When the host is genus Bacillus, SPO1 promoter, SPO2 promoter, penP promoter and the like are preferable.

When the host is yeast, PHO5 promoter, PGK promoter, GAP promoter, ADH promoter and the like are preferable.

When the host is insect cell, polyhedrin promoter, P10 promoter and the like are preferable.

As the expression vector, one containing, when desired, enhancer, splicing signal, poly A addition signal, selection marker, SV40 replication origin (hereinafter sometimes to be abbreviated as SV40 ori) and the like can be used besides those mentioned above. Examples of the selection marker include dihydrofolate reductase gene (hereinafter sometimes to be abbreviated as dhfr, methotrexate (MTX) resistance), ampicillin resistant gene (hereinafter sometimes to be abbreviated as amp^{r}), neomycin resistance gene (hereinafter sometimes to be abbreviated as neo^{r}, G418 resistance) and the like. Particularly, when dhfr gene deficient Chinese hamster cell is used, and dhfr gene is used as a selection marker, the object gene can also be selected in a medium free of thymidine.

Where necessary, a base sequence (signal codon) encoding a signal sequence suitable for the host may be added to the 5'-terminal side of a DNA encoding the fluorescent protein of the present invention (or substituted by a native signal codon). For example, when the host is the genus Escherichia, PhoA signal sequence, OmpA signal sequence and the like are used; and when the host is an animal cell, insulin signal sequence, α-interferon signal sequence, antibody molecule signal sequence and the like are used.

The fluorescent protein can be produced by transforming a host with an expression vector containing a DNA encoding the above-mentioned fluorescent protein, and culturing the obtained transformant.

As the host, for example, those suitable for expression such as genus Bacillus, yeast, insect cell, insect, animal cell, animal and the like are used.

As the genus Escherichia, for example, Escherichia coli K12·DH1, Escherichia coli JM103, Escherichia coli JA221, Escherichia coli HB101, Escherichia coli C600, Escherichia coli Hst08 and the like are used.

As the genus Bacillus, for example, Bacillus subtilis MI114, Bacillus subtilis 207-21 and the like are used.

As the yeast, for example, Saccharomyces cerevisiae AH22, AH22R⁻, NA87-11A, DKD-5D, 20B-12, Schizosaccharomyces pombe NCYC1913, NCYC2036, Pichia pastoris KM71 and the like are used.

As the insect cell, for example, when the virus is AcNPV, established lines of cells derived from Mamestra larva (Spodoptera frugiperda cell; Sf cell), MG1 cell derived from mid-intestine of Trichoplusia ni, High Five™ cell derived from egg of Trichoplusia ni, cell derived from Mamestra brassicae, cell derived from Estigmena acrea and the like are used. When the virus is BmNPV, as the insect cell, established lines of cells derived from silkworm (Bombyx mori N cell; BmN cell) and the like are used. As the Sf cell, Sf9 cell (ATCC CRL1711), Sf21 cell and the like are used.

As the insect, for example, Bombyx mori larva and the like are used.

As the animal cell, for example, cells such as COS-7, Vero, CHO, CHO (dhfr⁻), CHO-K1, L, AtT-20, GH3, FL, HEK293, NIH3T3, Balb3T3, FM3A, L929, SP2/0, P3U1, B16, P388 and the like are used.

As the animal, established transgenic mammal (e.g., mouse, rat, rabbit, sheep, swine, bovine), chicken and the like can be mentioned.

Transformation can be performed according to a known method according to the kind of the host.

The genus Escherichia can be transformed according to the methods described in, for example, Proc. Natl. Acad. Sci. USA, vol 69, 2110 (1972), Gene, vol. 17.,107 (1982) and the like.

The genus Bacillus can be transformed according to the method described in, for example, Molecular & General Genetics, vol. 168, 111 (1979) and the like.

The yeast can be transformed according to the method described in, for example, Methods in Enzymology, vol. 194, 182-187 (1991), Proc. Natl. Acad. Sci. USA, vol. 75, 1929 (1978) and the like.

The insect cell and insect can be transformed according to the method described in, for example, Bio/Technology, vol. 6, 47-55 (1988) and the like.

The animal cell can be transformed according to the method described in, for example, Cell Engineering, extra issue 8, New Cell Engineering Experimental Protocol, 263-267 (1995) (published by Shujunsha), Virology, vol. 52, 456 (1973).

The animal can be transformed according to the method described in, for example, development of transgenic animal (CMC Publishing), (2001).

The transformant can be cultured according to a known method according to the kind of the host.

For example, when a transformant whose host is the genus Escherichia is cultured, the medium is preferably M9 medium containing glucose and casamino acid. Where necessary, an agent such as 3β-indolylacrylic acid may be added to the medium to ensure efficient action of the promoter. The transformant is cultured at generally about 15 - about 43°C for about 3 - about 24 hr. Where necessary, aeration and stirring may be performed.

When a transformant whose host is the genus Bacillus is cultured, the medium is preferably a liquid medium. The medium preferably contains carbon source, nitrogen source, inorganic substance and the like necessary for the growth of the transformant. Examples of the carbon source include glucose, dextrin, soluble starch, sucrose and the like; examples of the nitrogen source include inorganic or organic substances such as ammonium salts, nitrate salts, corn steep liquor, peptone, casein, meat extract, soybean cake, potato extract and the like; and examples of the inorganic substance include calcium chloride, sodium dihydrogen phosphate, magnesium chloride and the like. In addition, the medium may contain yeast extract, vitamins, growth promoting factor and the like. The pH of the medium is preferably about 5 - about 8. The transformant is cultured generally at about 30 - about 40°C for about 6 - about 24 hr. Where necessary, aeration and stirring may be performed.

When a transformant whose host is a yeast is cultured, the medium is preferably a Burkholder minimum medium, SD medium containing 0.5% casamino acid or the like. The pH of the medium is preferably about 5 - about 8. The transformant is cultured generally at about 20 - about 35°C for about 24 - about 72 hr. Where necessary, aeration and stirring may be performed.

When a transformant whose host is an insect cell or insect is cultured, the medium is preferably a Grace's Insect Medium added with an additive such as inactivated 10% bovine serum and the like as appropriate, or the like. The pH of the medium is preferably about 6.2 - about 6.4. The transformant is cultured generally at about 27°C for about 3 - about 5 days. Where necessary, aeration and stirring may be performed.

When a transformant whose host is an animal cell is cultured, the medium is preferably minimum essential medium (MEM), Dulbecco's modified Eagle medium (DMEM), RPMI 1640 medium, 199 medium or the like, each containing about 5 - about 20% of fetal bovine serum. The pH of the medium is preferably about 6 - about 8. The transformant is cultured generally at about 30 - about 40°C for about 15 - about 60 hr. Where necessary, aeration and stirring may be performed.

When the host is an animal, a transgenic animal is obtained from the transfected fertilized egg by a conventional method, raised under general breeding conditions, and mammal milk and chicken egg are harvested.

As mentioned above, the fluorescent protein of the present invention can be produced intracellularly or extracellularly using the transformant.

The fluorescent protein of the present invention can be separated and purified by a method known per se from the culture obtained by culturing the aforementioned transformant.

For example, when the fluorescent protein of the present invention is extracted from cultivated bacteria or cytoplasm of the cell, a method including suspending fungi or cells collected from a culture by a known method in a suitable buffer, rupturing the fungi or cells by ultrasonication, lysozyme and/or freeze-thawing and the like, and obtaining a crude extract of a soluble protein by centrifugation, filtration and the like as appropriate. The buffer may contain protein denaturant such as urea, hydrochloric acid guanidine and the like, and surfactant such as TritonX-100™ and the like. When the fluorescent protein of the present invention is secreted out from the fungus (cell),
a method for separating the culture supernatant from the culture by centrifugation, filtration and the like, and the like is used.

The fluorescent protein of the present invention contained in the thus-obtained soluble fraction and culture supernatant can be isolated and purified by a method known per se. As such method, a method utilizing the solubility such as salting out, solvent precipitation and the like; a method mainly utilizing difference in the molecular weight such as dialysis, ultrafiltration, gel filtration method, and SDS-polyacrylamide gel electrophoresis and the like; a method utilizing difference in the electric charge such as ion exchange chromatography and the like; a method utilizing specific affinity such as affinity chromatography and the like; a method utilizing difference in the hydrophobicity such as reversed-phase high performance liquid chromatography and the like; a method utilizing difference in isoelectric point such as isoelectric focusing and the like; and the like are used. These methods can also be combined as appropriate.

The presence of the thus-obtained fluorescent protein of the present invention can be confirmed by enzyme immunoassay, Western blotting and the like, using an antibody to the fluorescent protein.

The present invention also provides a fusion protein comprising the fluorescent protein of the present invention and other protein (hereinafter to be also referred to as the fusion protein of the present invention). The method for acquiring the fusion protein of the present invention is not particularly limited, and the protein may be a chemically or biochemically synthesized protein, or a recombinant protein produced from a transformant, like the fluorescent protein of the present invention.

The fusion protein of the present invention may have any constitution as long as it contains the fluorescent protein of the present invention and other protein. For example, they may be fused in the order of the fluorescent protein of the present invention-other protein or other protein-the fluorescent protein of the present invention. The two proteins may be linked by a known suitable linker sequence. When the fusion protein of the present invention is produced from a transformant, it can be produced by linking a DNA fragment encoding the fusion protein of the present invention to the downstream of the promoter in a suitable expression vector, and transforming a suitable host cell, like the fluorescent protein of the present invention.

While the kind of other protein to be fused with the fluorescent protein of the present invention is not particularly limited, for example, a protein localized in cytoplasmic substrate, intracellular organelle (e.g., nucleus, endoplasmic reticulum, golgi apparatus, endosome, lysosome, mitochondria and the like), cellular membrane and the like, a protein forming a complex, targeting signal (e.g., nuclear localization signal, mitochondrial presequence) and the like are preferable.

Also, the fluorescent protein of the present invention can be used as a reporter protein for the measurement of promoter activity. That is, the activity of a test promoter can be measured by constructing a vector containing a DNA encoding the fluorescent protein of the present invention configured at the downstream of the test promoter, introducing the vector into a host cell, and detecting the fluorescence of the fluorescent protein of the present invention emitted from the cell. The test promoter is not particularly limited as long as it functions in the host cell.

The fusion protein of the present invention or an expression vector expressing the protein, obtained as mentioned above, is introduced into the cell and the position of the fluorescence in the cell is measured, whereby intracellular localization or dynamics of the fusion protein can be detected.

For example, using a protein specific to intracellular organelle as other protein to be fused with the fluorescent protein of the present invention, the position permitting detection of intracellular fluorescence is examined, whereby the distribution and movement of the protein in the nucleus, mitochondria, endoplasmic reticulum, golgi apparatus, secretion vesicle, peroxisome and the like can be observed.

Furthermore, for example, axon, dendrite and the like of nerve cell show markedly complicated variation in the running direction in an individual under development. Therefore, dynamic analysis can be performed by fluorescence labeling such moieties.

Alternatively, complex formation of a fusion protein can be detected by intracellularly introducing two or more, the same or different fusion proteins of the present invention or an expression vector expressing the protein, and detecting shift of the maximum fluorescence wavelength in the cell (maximum fluorescence wavelength becomes longer by about 5 nm - about 20 nm, preferably about 10 nm - about 15 nm, most preferably about 12 nm, as compared to monomer). In this method, complex formation of the fusion proteins can also be detected by observing two or more, the same or different fusion proteins of the present invention in a test tube and the like.

For example, complex formation of the fusion proteins in the cell or test tube can also be detected by examining whether the maximum fluorescence wavelength is elongated by using a protein constituting a complex as other protein to be fused with the fluorescent protein of the present invention.

The fluorescence of the fluorescent protein of the present invention can be detected in viable cells. Such detection can be performed using, for example, a fluorescence microscope (Olympus/IX71), an image analysis apparatus (Princeton Instruments/WinSpec 32) and the like. The kind of the microscope can be appropriately selected according to the object. When frequent observation is necessary for chasing time-course changes and the like, a general incident-light fluorescence microscope is preferable. When resolution is important such as when intracellular detailed localization is desired to be pursued and the like, a confocallaser microscope is preferable. As the microscope system, an inverted microscope is preferable from the aspects of maintenance of the physiological condition of the cell, and prevention of contamination. When an upright microscope with a high-magnification lens is used, a water immersion lens or oil immersion lens can be used.

As a filter set, an appropriate one can be selected according to the fluorescence wavelength of the fluorescent protein. When the fluorescent protein of the present invention is a monomer, the maximum excitation wavelength is 493 nm, and the maximum fluorescence wavelength is 513 nm. When it is a dimer, the maximum excitation wavelength is 510 nm, and the maximum fluorescence wavelength is 525 nm. Therefore, a filter with an excitation light of about 470-495 nm, and fluorescence of about 510-550 nm is preferably used.

When a time-course observation of viable cells is performed using a fluorescence microscope, a highly sensitive cooled CCD camera is preferably used for shooting in a short time. A cooled CCD camera reduces thermal noise by cooling CCD, and can shoot a feeble fluorescence image clearly by exposure for a short time.

According to the present invention, a kit for the analysis of localization of intracellular components and/or analysis of complex formation, which comprises at least one kind selected from the fluorescent protein, fusion protein, DNA, recombinant vector and transformant, described in the present specification, is provided. The kit of the present invention can be prepared using conventional materials and methods known per se.

Reagents such as fluorescent protein, DNA and the like can be prepared in a form suitable for preservation by dissolving them in a suitable solvent. As the solvent, water, ethanol, various buffers and the like can be used.

### Examples

The present invention is explained in more detail in the following by referring to Examples, which are not to be construed as limitative.

### Example 1 Production of mutant fluorescent protein expression vector

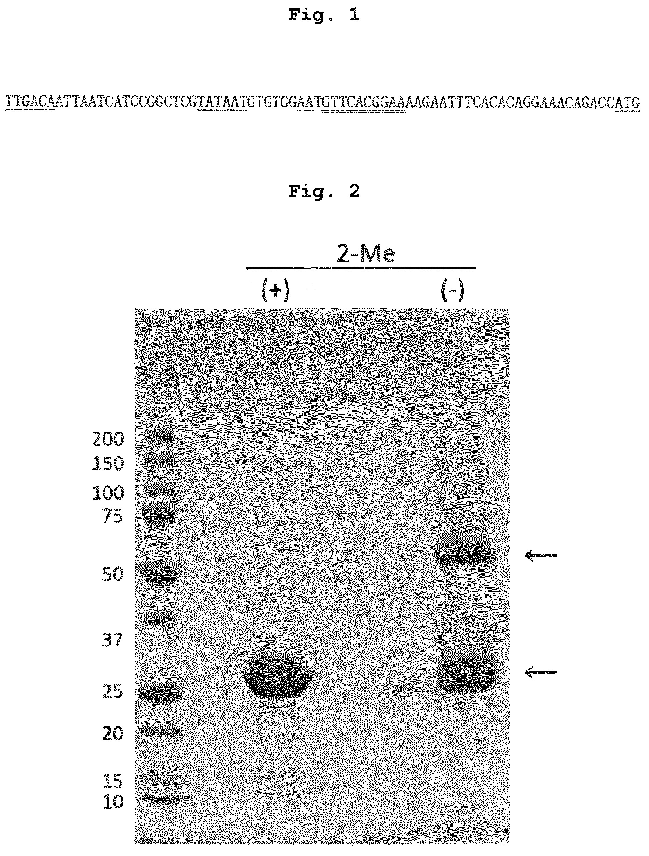

A plasmid having the GFP gene of the present invention (hereinafter B-maggio gene; SEQ ID NO: 1) (pTrc-do-b-maggio) was produced. First, a vector (pTrc-do-gfpuv) (SEQ ID NO: 3) wherein GFPuv gene was linked to pTrc-do vector which is a pTrc99A vector containing a modified promoter was produced. The pTrc-do promoter (Fig. 1; SEQ ID NO: 4) is a promoter modified to avoid binding of LacI by mutating 2 bases in the operator to which lac-derived LacI repressor binds. Using pTrc-do-gfpuv as a template and the following primer set, PCR was performed to amplify 4 DNA fragments containing mutations.
primer set 1 (gfp_C48S_R, gfp_Q204C_F)
primer set 2 (gfp_C48S_F, gfp_FS65LT_R)
primer set 3 (gfp_FS65LT_F, gfp_F99S_R)
primer set 4 (gfp_F99S_F, gfp_Q204C_R)

**Table 1**

| primer | base sequence |
|---|---|
| **gfp_C48S_R** | agtagtgctaataaatttaagggtaagttttcc (SEQ ID NO: 5) |
| **gfp_C48S_F** | aatttattagcactactggaaaactacctg (SEQ ID NO: 6) |
| **gfp_FS65LT_R** | catacgtcagagtagtgacaagtgttggcc (SEQ ID NO: 7) |
| **gfp_FS65LT_F** | tactctgacgtatggtgttcaatgcttttcccg (SEQ ID NO: 8) |
| **gfp_F99S_R** | ctttgaaaaatatagtgcgttcctgtac (SEQ ID NO: 9) |
| **gfp_F99S_F** | cactatatttttcaaagatgacgggaac (SEQ ID NO: 10) |
| **gfp_Q244C_R** | gcagagcatgtcgacaggtaatggttg (SEQ ID NO: 11) |
| **gfp_Q204C_F** | tcgacatgctctgccctttcgaaagatc (SEQ ID NO: 12) |

The amplified 4 fragments were confirmed by electrophoresis, and the object DNA fragments were purified.
The 4 DNA fragments were mixed, Escherichia coli HST08 was transformed by electroporation with a DNA fragment amplified by PCR using a primer set gfp_C48S_R and gfp_C48S_F, and selected using ampicillin. The obtained colonies mostly showed strong fluorescence as compared to known GFP.

### Example 2 In vitro photo-bleaching time of fluorescent protein

Each purified GFP (B-maggio (SEQ ID NO: 2), S65TGFP, Venus) solution (50 mM phosphate buffer (pH 7.2)) was sandwiched between glass plates (being OD<<0.05, shielding effect and diffusion due to self optical absorption can be ignored), and the quantity of light of the aforementioned GFP was measured at ambient temperature. As a fluorescence microscope, Olympus IX-71 provided with oil immersion objective lens of Olympus PlanApoUVx100 was used. As the mirror set, NIBF unit (excitation 475-495 nm, dichroic 505 nm, transmission 510-550 nm) was used. The quantity of light was 16 bit measured by EMCCD Princeton E2V96B and, using Image J and Kaleida Graph, the time-course changes of light quantity I(t) were fitted to I(t) = a+bt+cexp(-t/d) with convergence condition of residual change<1%, and a, b, c, d were determined, wherein a shows background light intensity, b shows time change by optical mechanical drift slower than quenching process, due to thermal expansion and the like, c shows maximum fluorescence intensity of GFP to be quenched (in proportion to molecule number in optical path), d shows photo-bleaching time (decay time). The obtained photo-bleaching time is shown in Table 2. It was found that B-maggio continuously shows 250 times longer fluorescence in vitro as compared to Venus.

**Table 2**

| GFP | decay time (s) | ratio |
|---|---|---|
| B-maggio | 202 ± 3 | 250 |
| S65TGFP | 0.15 ± 0.01 | 0.2 |
| Venus | 0.8 ± 0.4 | (1) |

| | | |
|---|---|---|
| photo-bleaching time of Venus set to 1 | | |

### Example 3 In vitro photo-bleaching time of fluorescent protein

A plasmid having each GFP (Venus, GFPUV, GFPUV4, Super Folder) gene in addition to the aforementioned pTrc-do-b-maggio was produced. Similar to B-maggio gene, the aforementioned GFP gene was linked to pTrc-do vector and designed to show gene expression. Escherichia coli Hst08 strain was transformed with the aforementioned plasmid, and the obtained each transformed strain was cultured in LB Lenox medium for 36 hr. The culture medium was brought into contact with an amino-coated slide glass (Matsunami MAS S9441) for 10 min, washed with water, sealed with a cover glass, and the light quantity was measured by the same photometry system as in Example 2 at ambient temperature. In addition, analysis was similarly performed as in Example 2. The obtained photo-bleaching time is shown in Table 3. It was found that B-maggio continuously shows 110 times longer fluorescence in vivo as compared to Venus.

**Table 3**

| GFP | bleaching time (s) | ratio |
|---|---|---|
| B-maggio | 700 ± 85 | 110 |
| Venus | 6.62 ± 0.03 | (1) |
| GFPUV | 5.9 ± 0.1 | 0.9 |
| GFPUV4 | 5.7 ± 1.2 | 0.9 |
| Super Folder | 4.6 ± 0.9 | 0.7 |

| | | |
|---|---|---|
| photo-bleaching time of Venus set to 1 | | |

### Example 4 Complex formation

The purified B-maggio was or was not treated with 2-mercaptoethanol (2-Me (+) or 2-Me(-)), separated by SDS-polyacrylamide gel electrophoresis, and the gel was stained (Fig. 2). As a result, proteins having two kinds of molecular weight (around 50 - 60 Kda, around 25 - 30 Kda) were confirmed in B-maggio (28.5 Kda) not treated with 2-mercaptoethanol. On the other hand, only a protein having one kind of molecular weight (around 25 - 30 Kda) was confirmed in B-maggio treated with 2-mercaptoethanol. Therefore, B-maggio was suggested to form a dimer with a disulfide bond.

### Example 5 Release of red fluorescence by dimer formation

It was confirmed that B-maggio has a peak at 493 nm as an excitation spectrum, and a peak at 513 nm as a fluorescence spectrum (Fig. 3). Green fluorescence could be easily observed using a B excitation mirror set and a fluorescence microscope. However, when B-maggio was observed using a G excitation mirror set (e.g., Olympus MWIG2, excitation 530-550 nm, 570 nm dichroic mirror, transmission around 575 nm), red fluorescence was scarcely observed. Similarly, when Escherichia coli strongly expressing B-maggio scarcely showed red fluorescence with the aforementioned G excitation mirror set. However, when Escherichia coli strongly expressing B-maggio was excited with B excitation light or V excitation light having a shorter wavelength and thereafter observed using the aforementioned G excitation mirror set, red fluorescence could be observed. Furthermore, when dithiothreitol (DTT), which is the same reducing agent as 2-mercaptoethanol, was added to the medium for culturing Escherichia coli strongly expressing B-maggio, red fluorescence could not be observed. Therefore, a possibility was suggested that B-maggio releases red fluorescence by exciting with B excitation light or V excitation light through dimer formation.

### Example 6 Transition of maximum fluorescence wavelength of dimer B-maggio

To verify the above-mentioned possibility, Twin B-maggio having 2 molecules of B-maggio linked by a linker sequence. To be specific, an expression vector expressing a dimer B-maggio wherein 10 residues on the C terminal of one of the B-maggios and 5 residues on the N terminal of the other B-maggio are substituted and linked by 20 residues of a linker sequence of tdTomato, which is a fluorescent protein having a dimer structure, was designed. Twin B-maggio showed a 12 nm longer maximum fluorescence wavelength than monomer B-maggio (Fig. 3). In addition, the relative amount of fluorescence at an observation wavelength (>575 nm: red) after excitation with G excitation light was higher for Twin B-maggio than for B-maggio. Being a dimer via a linker sequence, red fluorescence could be observed irrespective of the presence or absence of 20 mM 2-mercaptoethanol. From the above aspects, it was verified that monomer B-maggio releases red fluorescence through dimer formation.

### Example 7 Verification of B-maggio as highliter protein

It is known that bacteria form a dimer of 70S ribosome in the stationary phase. However, the dimer formation was detected solely by ultracentrifugation analysis, and could not be detected heretofore by one cell biology under a microscope. From the finding of structural biology (structure of 70S ribosome and GFP) accumulated till date, a possibility was considered that, when B-maggio is fused with the C-terminal of S10 protein of 70S ribosome, fused two B-maggios each take a spatial configuration similar to that of dimer formation through dimer formation of 70S ribosome (Fig. 5).

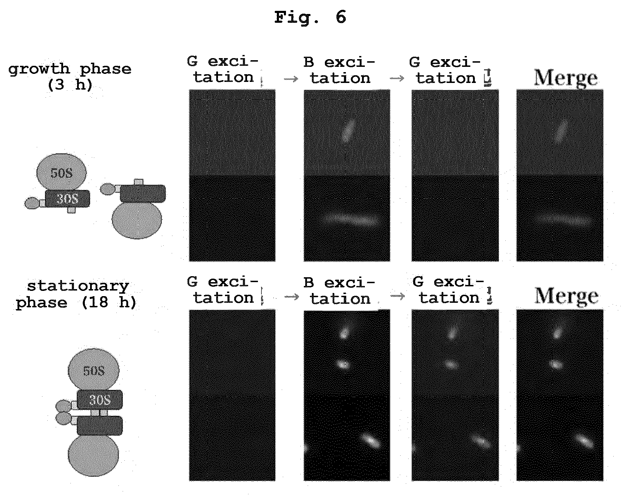

Thus, Escherichia coli mutation strain (S10-B-maggio strain) wherein B-maggio gene is inserted into chromosomal rpsJ gene (ribosome protein S10) and Escherichia coli mutation strain (S2-B-maggio strain) wherein B-maggio gene is inserted into chromosome rpsB gene (ribosome protein S2) were produced. While S10-B-maggio strain grew similarly as wild-type strain, S2-B-maggio strain died early since the stationary phase was shortened. Since 100S ribosome is not formed in the growth phase, red fluorescence could not be detected even when excitation with G excitation light was performed after excitation of S10-B-maggio strain with B excitation light (Fig. 6). However, structural biology suggests a possibility of formation of a dimer of B-maggio in the stationary phase when 100S ribosome formation takes place. In this stage, therefore, excitation of S10-B-maggio strain with G excitation light was performed after excitation with B excitation light, and the fluorescence of the cell suspension was measured to observe red fluorescence. Such fluorescence could not be observed even in the same period in the S2-B-maggio strain incapable of 100S ribosome formation, that underwent the same treatment. The red fluorescence essentially required excitation with B, V or U excitation light. That is, red fluorescence was observed in B-maggio with dimer formation since excitation with G excitation light was performed after excitation with B, V or U excitation light in advance. In addition, S10-B-maggio strain was cultured in LB medium, the cells in the time zone when ribosome forms 100S were collected, suspended in M9 medium, and observed at 1 mm x 1 mm cell. As a result, the relative amount of fluorescence at an observation wavelength (>575 nm) after excitation with G excitation light was higher than for S2-B-maggio strain incapable of forming 100S. Therefore, it was verified that monomer B-maggio releases red fluorescence through dimer formation also in the cells.

### Industrial Applicability

Using the fluorescent protein of the present invention, in vivo or in vitro observation of fluorescence in a sample can be facilitated. The fluorescence of the fluorescent protein can be observed with a standard B excitation mirror set. Furthermore, since the maximum fluorescence wavelength is elongated by 12 nm via dimer formation, dimer formation in the target molecule can be confirmed with ease.

This application is based on a patent application No. 2013-246642 filed in Japan (filing date: November 28, 2013), the contents of which are incorporated in full herein.

## Claims

1. A fluorescent protein comprising an amino acid sequence the same or substantially the same as the amino acid sequence shown in SEQ ID NO: 2.

2. A nucleic acid comprising a base sequence encoding the fluorescent protein according to claim 1.

3. An expression vector comprising the nucleic acid according to claim 2.

4. A transformant comprising the expression vector according to claim 3.

5. A fusion protein comprising the fluorescent protein according to claim 1 and other protein.

6. A method of detecting localization or dynamics of the fusion protein according to claim 5 in a cell, comprising measuring the position of fluorescence of the fusion protein in the cell.

7. A method of detecting formation of a complex of two or more, the same or different fusion proteins according to claim 5, comprising measuring the maximum fluorescence wavelength of the complex.


SEQUENCE LISTING

<110>  HORIBA, LTD.
       KYOTO SANGYO UNIVERSITY

<120>  novel fluorescent protein

<130>  HORP13049

<150>  JP 2013-246642
<151>  2013-11-28

<160>  12

<170>  PatentIn version 3.5

<210>  1
<211>  717
<212>  DNA
<213>  Aequorea victoria

<220>
<221>  CDS
<222>  (1)..(717)

<400>  1
atg agt aaa gga gaa gaa ctt ttc act gga gtt gtc cca att ctt gtt        48
Met Ser Lys Gly Glu Glu Leu Phe Thr Gly Val Val Pro Ile Leu Val
1               5                   10                  15

gaa tta gat ggt gat gtt aat ggg cac aaa ttt tct gtc agt gga gag        96
Glu Leu Asp Gly Asp Val Asn Gly His Lys Phe Ser Val Ser Gly Glu
            20                  25                  30

ggt gaa ggt gat gca aca tac gga aaa ctt acc ctt aaa ttt att agc       144
Gly Glu Gly Asp Ala Thr Tyr Gly Lys Leu Thr Leu Lys Phe Ile Ser
        35                  40                  45

act act gga aaa cta cct gtt cca tgg cca aca ctt gtc act act ctg       192
Thr Thr Gly Lys Leu Pro Val Pro Trp Pro Thr Leu Val Thr Thr Leu
    50                  55                  60

acg tat ggt gtt caa tgc ttt tcc cgt tat ccg gat cat atg aaa cgg       240
Thr Tyr Gly Val Gln Cys Phe Ser Arg Tyr Pro Asp His Met Lys Arg
65                  70                  75                  80

cat gac ttt ttc aag agt gcc atg ccc gaa ggt tat gta cag gaa cgc       288
His Asp Phe Phe Lys Ser Ala Met Pro Glu Gly Tyr Val Gln Glu Arg
                85                  90                  95

act ata ttt ttc aaa gat gac ggg aac tac aag acg cgt gct gaa gtc       336
Thr Ile Phe Phe Lys Asp Asp Gly Asn Tyr Lys Thr Arg Ala Glu Val
            100                 105                 110

aag ttt gaa ggt gat acc ctt gtt aat cgt atc gag tta aaa ggt att       384
Lys Phe Glu Gly Asp Thr Leu Val Asn Arg Ile Glu Leu Lys Gly Ile
        115                 120                 125

gat ttt aaa gaa gat gga aac att ctc gga cac aaa ctc gag tac aac       432
Asp Phe Lys Glu Asp Gly Asn Ile Leu Gly His Lys Leu Glu Tyr Asn
    130                 135                 140

tat aac tca cac aat gta tac atc acg gca gac aaa caa aag aat gga     480
Tyr Asn Ser His Asn Val Tyr Ile Thr Ala Asp Lys Gln Lys Asn Gly
145                 150                 155                 160

atc aaa gct aac ttc aaa att cgc cac aac att gaa gat gga tcc gtt     528
Ile Lys Ala Asn Phe Lys Ile Arg His Asn Ile Glu Asp Gly Ser Val
                165                 170                 175

caa cta gca gac cat tat caa caa aat act cca att ggc gat ggc cct     576
Gln Leu Ala Asp His Tyr Gln Gln Asn Thr Pro Ile Gly Asp Gly Pro
            180                 185                 190

gtc ctt tta cca gac aac cat tac ctg tcg aca tgc tct gcc ctt tcg     624
Val Leu Leu Pro Asp Asn His Tyr Leu Ser Thr Cys Ser Ala Leu Ser
        195                 200                 205

aaa gat ccc aac gaa aag cgt gac cac atg gtc ctt ctt gag ttt gta     672
Lys Asp Pro Asn Glu Lys Arg Asp His Met Val Leu Leu Glu Phe Val
    210                 215                 220

act gct gct ggg att aca cat ggc atg gat gag ctc tac aaa taa         717
Thr Ala Ala Gly Ile Thr His Gly Met Asp Glu Leu Tyr Lys
225                 230                 235


<210>  2
<211>  238
<212>  PRT
<213>  Aequorea victoria

<400>  2

Met Ser Lys Gly Glu Glu Leu Phe Thr Gly Val Val Pro Ile Leu Val
1               5                   10                  15


Glu Leu Asp Gly Asp Val Asn Gly His Lys Phe Ser Val Ser Gly Glu
            20                  25                  30


Gly Glu Gly Asp Ala Thr Tyr Gly Lys Leu Thr Leu Lys Phe Ile Ser
        35                  40                  45


Thr Thr Gly Lys Leu Pro Val Pro Trp Pro Thr Leu Val Thr Thr Leu
    50                  55                  60


Thr Tyr Gly Val Gln Cys Phe Ser Arg Tyr Pro Asp His Met Lys Arg
65                  70                  75                  80


His Asp Phe Phe Lys Ser Ala Met Pro Glu Gly Tyr Val Gln Glu Arg
                85                  90                  95


Thr Ile Phe Phe Lys Asp Asp Gly Asn Tyr Lys Thr Arg Ala Glu Val
            100                 105                 110


Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Asn Ser His Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Cys Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr His Gly Met Asp Glu Leu Tyr Lys
225                 230                 235

<210>  3
<211>  4831
<212>  DNA
<213>  Artificial Sequence

<220>
<223>  gfpuv expression vector

<400>  3
gtttgacagc ttatcatcga ctgcacggtg caccaatgct tctggcgtca ggcagccatc      60

ggaagctgtg gtatggctgt gcaggtcgta aatcactgca taattcgtgt cgctcaaggc     120

gcactcccgt tctggataat gttttttgcg ccgacatcat aacggttctg gcaaatattc     180

tgaaatgagc tgttgacaat taatcatccg gctcgtataa tgtgtggaat gttcacggaa     240

aagaatttca cacaggaaac agaccatgag taaaggagaa gaacttttca ctggagttgt     300

cccaattctt gttgaattag atggtgatgt taatgggcac aaattttctg tcagtggaga     360

gggtgaaggt gatgcaacat acggaaaact tacccttaaa tttatttgca ctactggaaa     420

actacctgtt ccatggccaa cacttgtcac tactttctct tatggtgttc aatgcttttc     480

ccgttatccg gatcatatga aacggcatga ctttttcaag agtgccatgc ccgaaggtta     540

tgtacaggaa cgcactatat ctttcaaaga tgacgggaac tacaagacgc gtgctgaagt     600

caagtttgaa ggtgataccc ttgttaatcg tatcgagtta aaaggtattg attttaaaga     660

agatggaaac attctcggac acaaactcga gtacaactat aactcacaca atgtatacat     720

cacggcagac aaacaaaaga atggaatcaa agctaacttc aaaattcgcc acaacattga     780

agatggatcc gttcaactag cagaccatta tcaacaaaat actccaattg gcgatggccc 840
tgtcctttta ccagacaacc attacctgtc gacacaatct gccctttcga aagatcccaa 900
cgaaaagcgt gaccacatgg tccttcttga gtttgtaact gctgctggga ttacacatgg 960
catggatgag ctctacaaat aagctgtttt ggcggatgag agaagatttt cagcctgata 1020
cagattaaat cagaacgcag aagcggtctg ataaaacaga atttgcctgg cggcagtagc 1080
gcggtggtcc cacctgaccc catgccgaac tcagaagtga aacgccgtag cgccgatggt 1140
agtgtggggt ctccccatgc gagagtaggg aactgccagg catcaaataa aacgaaaggc 1200
tcagtcgaaa gactgggcct ttcgttttat ctgttgtttg tcggtgaacg ctctcctgag 1260
taggacaaat ccgccgggag cggatttgaa cgttgcgaag caacggcccg gagggtggcg 1320
ggcaggacgc ccgccataaa ctgccaggca tcaaattaag cagaaggcca tcctgacgga 1380
tggccttttt gcgtttctac aaactctttt tgtttatttt tctaaataca ttcaaatatg 1440
tatccgctca tgagacaata accctgataa atgcttcaat aatattgaaa aaggaagagt 1500
atgagtattc aacatttccg tgtcgccctt attccctttt ttgcggcatt ttgccttcct 1560
gtttttgctc acccagaaac gctggtgaaa gtaaaagatg ctgaagatca gttgggtgca 1620
cgagtgggtt acatcgaact ggatctcaac agcggtaaga tccttgagag ttttcgcccc 1680
gaagaacgtt ttccaatgat gagcactttt aaagttctgc tatgtggcgc ggtattatcc 1740
cgtgttgacg ccgggcaaga gcaactcggt cgccgcatac actattctca gaatgacttg 1800
gttgagtact caccagtcac agaaaagcat cttacggatg gcatgacagt aagagaatta 1860
tgcagtgctg ccataaccat gagtgataac actgcggcca acttacttct gacaacgatc 1920
ggaggaccga aggagctaac cgcttttttg cacaacatgg gggatcatgt aactcgcctt 1980
gatcgttggg aaccggagct gaatgaagcc ataccaaacg acgagcgtga caccacgatg 2040
cctacagcaa tggcaacaac gttgcgcaaa ctattaactg gcgaactact tactctagct 2100
tcccggcaac aattaataga ctggatggag gcggataaag ttgcaggacc acttctgcgc 2160
tcggcccttc cggctggctg gtttattgct gataaatctg gagccggtga gcgtgggtct 2220
cgcggtatca ttgcagcact ggggccagat ggtaagccct cccgtatcgt agttatctac 2280
acgacgggga gtcaggcaac tatggatgaa cgaaatagac agatcgctga gataggtgcc 2340
tcactgatta agcattggta actgtcagac caagtttact catatatact ttagattgat 2400
ttaaaacttc atttttaatt taaaaggatc taggtgaaga tcctttttga taatctcatg 2460
accaaaatcc cttaacgtga gttttcgttc cactgagcgt cagaccccgt agaaaagatc 2520
aaaggatctt cttgagatcc tttttttctg cgcgtaatct gctgcttgca aacaaaaaaa 2580
ccaccgctac cagcggtggt ttgtttgccg gatcaagagc taccaactct ttttccgaag 2640

gtaactggct tcagcagagc gcagatacca aatactgtcc ttctagtgta gccgtagtta 2700

ggccaccact tcaagaactc tgtagcaccg cctacatacc tcgctctgct aatcctgtta 2760

ccagtggctg ctgccagtgg cgataagtcg tgtcttaccg ggttggactc aagacgatag 2820

ttaccggata aggcgcagcg gtcgggctga acggggggtt cgtgcacaca gcccagcttg 2880

gagcgaacga cctacaccga actgagatac ctacagcgtg agctatgaga aagcgccacg 2940

cttcccgaag ggagaaaggc ggacaggtat ccggtaagcg gcagggtcgg aacaggagag 3000

cgcacgaggg agcttccagg gggaaacgcc tggtatcttt atagtcctgt cgggtttcgc 3060

cacctctgac ttgagcgtcg atttttgtga tgctcgtcag gggggcggag cctatggaaa 3120

aacgccagca acgcggcctt tttacggttc ctggcctttt gctggccttt tgctcacatg 3180

ttctttcctg cgttatcccc tgattctgtg gataaccgta ttaccgcctt tgagtgagct 3240

gataccgctc gccgcagccg aacgaccgag cgcagcgagt cagtgagcga ggaagcggaa 3300

gagcgcctga tgcggtattt tctccttacg catctgtgcg gtatttcaca ccgcatatgg 3360

tgcactctca gtacaatctg ctctgatgcc gcatagttaa gccagtatac actccgctat 3420

cgctacgtga ctgggtcatg gctgcgcccc gacacccgcc aacacccgct gacgcgccct 3480

gacgggcttg tctgctcccg gcatccgctt acagacaagc tgtgaccgtc tccgggagct 3540

gcatgtgtca gaggttttca ccgtcatcac cgaaacgcgc gaggcagcag atcaattcgc 3600

gcgcgaaggc gaagcggcat gcatttacgt tgacaccatc gaatggtgca aaacctttcg 3660

cggtatggca tgatagcgcc cggaagagag tcaattcagg gtggtgaatg tgaaaccagt 3720

aacgttatac gatgtcgcag agtatgccgg tgtctcttat cagaccgttt cccgcgtggt 3780

gaaccaggcc agccacgttt ctgcgaaaac gcgggaaaaa gtggaagcgg cgatggcgga 3840

gctgaattac attcccaacc gcgtggcaca acaactggcg ggcaaacagt cgttgctgat 3900

tggcgttgcc acctccagtc tggccctgca cgcgccgtcg caaattgtcg cggcgattaa 3960

atctcgcgcc gatcaactgg gtgccagcgt ggtggtgtcg atggtagaac gaagcggcgt 4020

cgaagcctgt aaagcggcgg tgcacaatct tctcgcgcaa cgcgtcagtg ggctgatcat 4080

taactatccg ctggatgacc aggatgccat tgctgtggaa gctgcctgca ctaatgttcc 4140

ggcgttattt cttgatgtct ctgaccagac acccatcaac agtattattt tctcccatga 4200

agacggtacg cgactgggcg tggagcatct ggtcgcattg ggtcaccagc aaatcgcgct 4260

gttagcgggc ccattaagtt ctgtctcggc gcgtctgcgt ctggctggct ggcataaata 4320

tctcactcgc aatcaaattc agccgatagc ggaacgggaa ggcgactgga gtgccatgtc 4380

cggttttcaa caaaccatgc aaatgctgaa tgagggcatc gttcccactg cgatgctggt 4440

tgccaacgat cagatggcgc tgggcgcaat gcgcgccatt accgagtccg ggctgcgcgt 4500

tggtgcggat atctcggtag tgggatacga cgataccgaa gacagctcat gttatatccc 4560

gccgttaacc accatcaaac aggattttcg cctgctgggg caaaccagcg tggaccgctt    4620

gctgcaactc tctcagggcc aggcggtgaa gggcaatcag ctgttgcccg tctcactggt    4680

gaaaagaaaa accaccctgg cgcccaatac gcaaaccgcc tctccccgcg cgttggccga    4740

ttcattaatg cagctggcac gacaggtttc ccgactggaa agcgggcagt gagcgcaacg    4800

caattaatgt gagttagcgc gaattgatct g                                   4831

<210>  4
<211>  76
<212>  DNA
<213>  Artificial Sequence

<220>
<223>  promoter

<400>  4
ttgacaatta atcatccggc tcgtataatg tgtggaatgt tcacggaaaa gaatttcaca      60

caggaaacag accatg                                                      76

<210>  5
<211>  33
<212>  DNA
<213>  Artificial Sequence

<220>
<223>  primer

<400>  5
agtagtgcta ataaatttaa gggtaagttt tcc                                   33

<210>  6
<211>  30
<212>  DNA
<213>  Artificial Sequence

<220>
<223>  primer

<400>  6
aatttattag cactactgga aaactacctg                                       30

<210>  7
<211>  30
<212>  DNA
<213>  Artificial Sequence

<220>
<223>  primer

<400>  7
catacgtcag agtagtgaca agtgttggcc                                       30

<210>  8

<211>  33
<212>  DNA
<213>  Artificial Sequence

<220>
<223>  primer

<400>  8
tactctgacg tatggtgttc aatgcttttc ccg                                    33


<210>  9
<211>  28
<212>  DNA
<213>  Artificial Sequence

<220>
<223>  primer

<400>  9
ctttgaaaaa tatagtgcgt tcctgtac                                          28


<210>  10
<211>  28
<212>  DNA
<213>  Artificial Sequence

<220>
<223>  primer

<400>  10
cactatattt ttcaaagatg acgggaac                                          28


<210>  11
<211>  27
<212>  DNA
<213>  Artificial Sequence

<220>
<223>  primer

<400>  11
gcagagcatg tcgacaggta atggttg                                           27


<210>  12
<211>  28
<212>  DNA
<213>  Artificial Sequence

<220>
<223>  primer

<400>  12
tcgacatgct ctgccctttc gaaagatc                                          28